# EUROPEAN PATENT SPECIFICATION

(11) **EP 2 047 269 B1**
(45) Date of publication and mention of the grant of the patent: **30.04.2014**
(21) Application number: 07766496.9
(22) Date of filing: 13.07.2007
(51) Int. Cl.: G01N 33/564

(54) **MEASUREMENT OF COMPLEMENT ACTIVATION PRODUCTS ON ANTIGEN MICROARRAYS**
MESSUNG VON KOMPLEMENTAKTIVIERUNGSPRODUKTEN AUF ANTIGEN-MIKROARRAYS
MESURE DES PRODUITS D'ACTIVATION DU COMPLÉMENT SUR DES ENSEMBLES D'ANTIGÈNES PAR TECHNOLOGIE MICROARRAY

(30) Priority: 14.07.2006 US 830558 P
(43) Date of publication of application: 15.04.2009
(73) Proprietor: Eötvös Lorand University, 1053 Budapest (HU); Hungarian Academy Of Sciences TKI, 1067 Budapest (HU)
(72) Inventor: PRECHL, József, H-2151 Fót (HU); PAPP, Krisztián, H-1065 Budapest (HU)
(74) Representative: Chapman, Paul William
(86) International application number: PCT/HU2007/000064
(87) International publication number: WO 2008/007159

(56) References cited:
- WO-A-2005/078442
- WAHRMANN M ET AL: "[C4d]FlowPRA Screening-A Specific Assay for Selective Detection of Complement-Activating Anti-HLA Alloantibodies" HUMAN IMMUNOLOGY, NEW YORK, NY, US, vol. 66, no. 5, May 2005 (2005-05), pages 526-534, XP004915120 ISSN: 0198-8859
- PAPP K ET AL: "On-chip complement activation adds an extra dimension to antigen microarrays" MOLECULAR AND CELLULAR PROTEOMICS, AMERICAN SOCIETY FOR BIOCHEMISTRY AND MOLECULAR BIOLOGY, US, vol. 6, no. 1, January 2007 (2007-01), pages 133-140, XP008086270 ISSN: 1535-9476
- THIRUMALAPURA ET AL: "Bacterial cell microarrays for the detection and characterization of antibodies against surface antigens", JOURNAL OF IMMUNOLOGICAL METHODS, ELSEVIER SCIENCE PUBLISHERS B.V.,AMSTERDAM, NL, vol. 309, no. 1-2, 20 February 2006 (2006-02-20), pages 48-54, XP005282598, ISSN: 0022-1759, DOI: DOI:10.1016/J.JIM.2005.11.016

## Description

The present invention relates to multiplex immunoassays utilizing antigen micro arrays, and more particularly to methods for qualitative and quantitative detection of antigens-specific complement activation in a biological sample, via the measurement of complement components deposited on antigen micro arrays.

The object of this invention is a method for the qualitative and quantitative analysis of complement activation on an antigen array,
wherein the antigen array comprises at least two antigens, and wherein covalently bound C3 and/or C4 fragments are detected with a labelled detecting agent, characterised by the following steps:
- using an antigen microarray as the antigen array, wherein antigens and reference materials are printed side-by-side, as spots on the microarray, at high spatial density;
- contacting the antigen microarray with a sample containing both at least one molecule that may be captured by the antigens disposed on the antigen microarray and a functional complement system;
- allowing complement activation to take place in a single reaction chamber per sample on the microarray, resulting in localized, antigen-specific deposition of C3 and/or C4 fragments;
- measuring bound C3 and/or C4 fragments covalently bound to the microarray as a result of complement activation by that particular antigens using a labelled detecting agent.
The antigens of the antigen array are preferably parasites, microbes, viruses, prions or components thereof.

The further subject of the present invention is, that antigens are selected from the group consisting of proteins, peptides, glycoproteins, lipoproteins, lipids, glycolipids, nucleic acids, carbohydrates, small molecules, and allergens or autoantigens.

The subject of the present invention is furthermore, that the sample includes a clinical sample, serum, plasma, biological fluid, and cultured cells, cell supernatants, cell lysates, or a pure or enriched sample derived from any of these.

The object of the present invention is furthermore, that the detecting agent is an antibody or portions or fragments thereof, or a soluble receptor protein or portions or fragments thereof, preferably a scaffold protein, nucleic acid aptamer or other affinity reagent obtained from libraries by molecular evolution and affinity screening.

The object of the present invention is furthermore, that detecting agent is labeled, functionally active C3 or C4 molecules, capable of covalently binding to the antigens on the array.

The object of the present invention is furthermore that the micro array is a two dimensional array on a solid surface.

The object of the present invention is furthermore that the micro array contains reference materials that capture complement C3 or C4 molecules, activate the complement system or are purified C3 or C4 proteins themselves.

The object of the present invention is furthermore a method for measurement of antibody dependent complement activation by an autoantigen or for comparison of control and autoimmune serum profiles, or for representation of concurrent C3 deposition and Ig binding measurements, or for comparison of C3 and C4 deposition patterns, comprising the following steps:

Low density nitrocellulose arrays comprising the indicated antigens were contacted with serum samples and kept in a humidified atmosphere at 37 degrees Celsius, then the arrays were subsequently washed in buffered saline solution, then fluorescently labeled C3 or C4 specific antibody, diluted to give optimal signal to noise ratio, was contacted with the arrays, agitated, then the array was washed again and scanned on a scanner to give an array where the at least one antigen that captured the molecule capable of complement activation is detected.

Where not otherwise stated the following definitions and abbreviations are used further on:

Antibody = immunoglobulin: any molecule or any class of immunoglobulin molecules, irrespective of their ability to activate complement with other words a heterotetrameric glycoprotein, which serves as the recognition molecule of the adaptive immune system. Antibodies comprise two heavy and two light chains. The antigen recognition site (Fv region, CDR or hypervariable region) is enormously diverse, with tens of millions of different specificities. The constant regions (C-region) also show modest variability due to protein sequence differences and carbohydrate content (glycosylation).

Antigen: any entity that is recognized by one of the various recognition molecules of the immune system. Antigens could be proteins, peptides, antibodies, glycoproteins, lipoproteins, glycolipids, small molecules, lipids, carbohydrates, nucleic acids, and allergens. Antigens could be whole eukaryotic or prokaryotic cells, viruses or prions and their derivatives. An antigen may be in its pure form or in a sample in which the antigen is mixed with other components.

Antigen microarray: a linear, or oligodimensional array of two or more different antigens formed in an indexible manner either on the surface of a solid support or on the surface of an array of solid supports (addressable beads) including many different agents (proteins, nucleic acids, lipids and related macromolecules) arrayed at high spatial density in an indexible manner on a solid support. Feature is a technical term describing a printed element or spot of the array.

Autoantibody: an antibody recognizing self structures; its presence in serum is characteristic for autoimmune diseases.

Complement: a collection of membrane bound and soluble proteins, part of the innate immune system, which function as recognition and effector molecules.

Complement deposition = complement fixation: a highly reactive chemical bond, a thioester bond, exposed upon activation of C3 or C4, covalently couples the complement component to a nearby molecule.

Detectably labeled: as used herein is intended to encompass detecting agent directly coupled to a detectable substance, such as a fluorescent dye, and detecting agent coupled to a member of binding pair, such as biotin/streptavidin, or an epitope tag or oligonucleotide that can specifically interact with a molecule that can be detected, such as by producing a colored substrate or fluorescence.

Detecting agent: any molecule that has the ability to selectively bind to immobilized complement C3 or C4 molecules or fragments. The detecting agent includes proteins (complement receptors), peptides (complement receptor fragments), and polyclonal, monoclonal or recombinant antibodies that specifically recognize C3 or C4. Affinity reagents like scaffold proteins, aptamers selected from libraries are also included. The detecting agent also includes directly labeled functional C3 or C4 molecules themselves.

Isotype: classification of antibodies based on the heavy chain sequence. Immunoglobulins are made in several distinct isotypes or classes-IgM, IgG, IgD, IgA, and IgE-each of which has a distinct heavy-chain C region encoded by a distinct C-region gene. The isotype of an antibody determines the effector mechanisms that it can engage on binding antigen.

Prion = proteinaceous infectious particle: a type of infectious agent composed only of protein.

Sample: a variety of sample types and/or origins, such as blood and other liquid samples of biological origin. It is the source both for antigen recognition molecules and complement, a clinical blood or serum sample, and also includes cell supernatants, cell lysates, plasma, and other bodily biological fluid, e.g. synovial fluid, mucosal secretion. These samples can be obtained and prepared by methods known in the art with the restriction that functionality of the complement system be preserved.

Zymosan: a cell wall component of yeast, activating complement via the lectin and alternative pathway.
Ab: antibody
Ag: antigen
Al(OH)₃: aluminium hydroxide
Alexa-647: fluorescent dye
a-mC3: goat anti-mouse complement C3 capture antibody
a-mIgG: goat anti-mouse IgG capture antibody
BSA: bovine serum albumin, negative control
C3: complement component C3
C4: complement component C4
Clq: complement protein C1q, part of the C1 complex
CCC: complement convertase complex
C3b: degradation product generated from C3 by CCC
C3bBb: C3 convertase of the alternative pathway
DNA: deoxyribonucleic acid, double stranded
dsDNA: double-stranded deoxyribonucleic acid
FITC: fluorescein isothiocyanate fluorescent dye
Ig: immunoglobulin
IgM: antibodies characterised by mu heavy chain
IgG: antibodies characterised by gamma heavy chain
IgD: antibodies characterised by delta heavy chain
IgA: antibodies characterised by alpha heavy chain
IgE: antibodies characterised by epsilon heavy chain
MBL: mannose binding lectin
PBS: phosphate buffered saline solution
pLA: bacterial superantigen fusion protein which activates complement via antibody binding, comprising protein A and protein L
RCA: the signals of detecting agents may be significantly increased by Rolling Circle Amplification.
SLE: systemic lupus erythematosus
TNP-BSA: Tri-nitro-phenol conjugated to Bovine Serum Albumin
TNP-KLH: Tri-nitro-phenol conjugated to Keyhole Limpet Hemocyanin

Microarray technology has become a crucial tool for large-scale and high-throughput biology. An antigen microarray comprises many different agents (proteins, nucleic acids, lipids and related macromolecules) arrayed at high spatial density on a solid support. Antigens are specifically recognized by antibodies and other serum components that are subsequently detected and quantified. The antigen microarray format enables fast, easy and parallel detection of thousands of addressable antigens and side-by-side measurements. It may be applied to analyze the spectrum of autoantigens recognized by autoantibodies, to characterize both pathogens and the evoked response during infections or following immunizations.

Currently, the captured serum components in an antigen array are detected by an indirect labeling technique in which bound proteins - most often antibodies - are detected using a secondary labeled antibody. These methods serve for identifying a collection of circulating serum antibodies against the arrayed antigens, therefore the techniques are also called antibody profiling (reviewed in W. H. Robinson., Curr. Opinion Chem. Biol., 2006, 10:67-72). Antibodies are categorized into classes (IgD, IgM, IgG, IgA, IgE) and subclasses (e.g. IgG1, IgG2, IgG3) which have distinct structures and immune functions. In addition to binding to their specific target antigen, they exert their effects by interacting with other components of the immune system, such as various cell types and the complement system. The identification of these subclasses therefore is of interest (Graham KL, Vaysberg M, Kuo A, Utz PJ.; Proteomics. 2006 Nov;6(21):5720-4). Importantly, different classes and subclasses of antibodies exert different effects, which are not addressed by current multiplex immunoprofiling assays.

Therefore, there is a need to have a system and method for the functional characterization of antigen-serum interactions, detecting not only antibody binding but also its consequences. This invention satisfies this need by disclosing a system and method of detecting the activation of the complement system by the elements of the antigen array. Other advantages of this invention will be apparent with reference to the detailed description.

The complement system is a network of soluble and membrane bound proteins. Activation of the system by one of the three described pathways (classical, lectin and alternative) results in the consecutive cleavage of proenzymes, generating active enzymes which cleave the next component of the cascade. The classical pathway can be initiated by antigen bound antibodies, and other molecules that bind C1q, like C-reactive protein or serum amyloid P protein. The lectin pathway relies on the mannose binding lectin (MBL) and ficolins, molecules that recognize carbohydrate patterns. The alternative pathway is capable of autoactivation, therefore surfaces that lack or are unable to bind complement regulatory proteins promote its activation. Importantly, all three pathways converge at the point of complement C3 cleavage and activation, a point where an amplification loop insures the generation of sufficient amounts of C3 fragments. Complement component C3 is additionally one of the most abundant serum proteins after albumin and immunoglobulins. C4 component cleavage is part of the classical and lectin pathway activation.

Two components of these generated cleavage fragments, complement C3 and C4, expose a highly reactive chemical bond, a thioester bond, which covalently couples the complement component to a nearby molecule. The covalently bound fragments mark the molecule for identification by the immune system, and thereby modulate the immune response against the antigen initiating complement activation. This covalent binding of complement C3 and C4 is also termed as complement deposition or complement fixing.

Antibodies can initiate and maintain complement activation by any of the three pathways exclusively when bound to antigen and with an efficiency that depends on their isotype, affinity and glycosylation.

The object of this invention provides a method for identifying antigens that can activate the complement system in a certain biological sample. Activation of the complement system can be by any of the known pathways, that is, classical, lectin or alternative pathway. The result of the activation is the generation of reactive complement C3 and C4 fragments - C3b and C4b - which form covalent bonds with the activating molecules. The method comprises the following steps of

A, providing the antigen array having at least two antigens, and control materials required for assessing complement function and used for normalization of data

B, contacting the antigen array - under appropriate conditions - with a sample containing molecules capable of activating a complement system and a functional complement system that allows deposition of complement C3 and C4,

C, and detecting the bound complement fragments with a detecting agent.

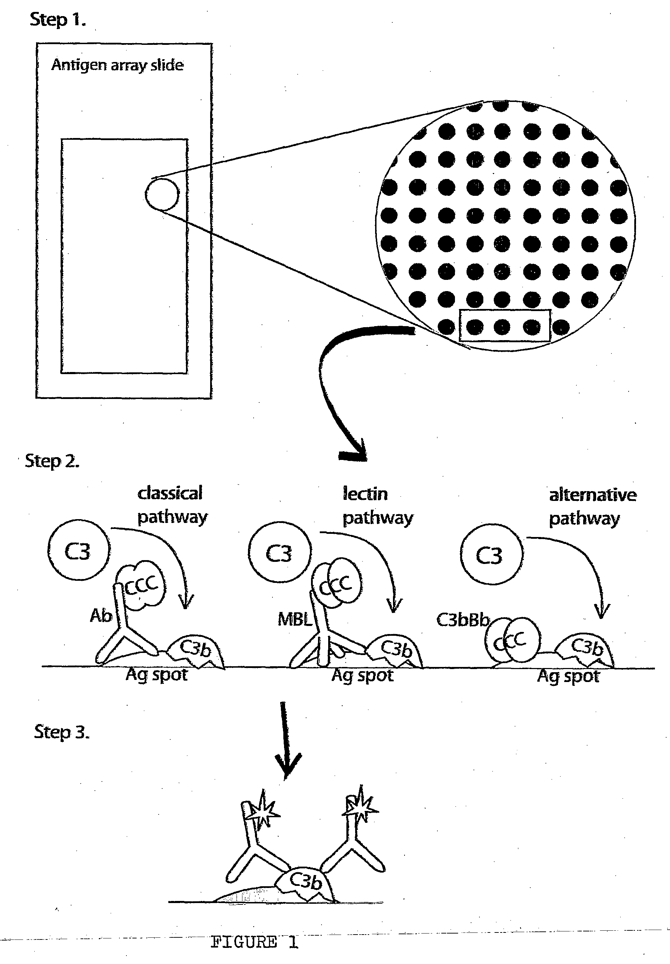

Figure 1. shows a schematic diagram of the measurement of complement deposition on antigen arrays.

Accordingly, one object of the present invention is to provide methods for their applications in clinical diagnostics and serum profiling, wherein the methods are highly specific and sensitive, and easily adapted to automation. By adding an extra dimension to the so far used antibody measurements, antigen arrays can provide more detailed information about the immunity of the tested individual. This information can be used for establishing diagnosis, differential diagnosis, disease staging with more accuracy. More detailed immunoprofiles also help the design of personalized medical treatment.

The objectives and advantages of the invention will become apparent from the following detailed description of preferred embodiments thereof.

The practice of the present invention will employ conventional techniques of molecular biology (including recombinant techniques), microbiology, cell biology, biochemistry and immunology, which are known to those skilled in the art. For example, monoclonal and polyclonal antibodies can be produced against C3 or C4 in different hosts by known methods. The manipulation of the antibodies is also known. In addition, the production and preparation of antigens are also known by expert skilled in the art. For the sake of brevity, no citation or detailed description of the known techniques will be given herein.

It is common that activation of the complement system by any of the three known pathways (lectin dependent, classical, alternative) converges at the point of C3 cleavage (see figure on this page). Thus, whichever pathway is activated, reactive C3b fragments will be generated and will bind to any neighbouring molecules in the vicinity of activation. Complement component C4 is cleaved when the classical or the lectin mediated pathways are activated, thus covalently bound C4 fragments will be generated only when these aforementioned pathways are active. Due to the presence of an amplification loop generating C3b, large numbers of this molecule can be produced by a single activating entity (summarised on the following flow sheet). This phenomenon renders C3b detection a potentially very sensitive technology.

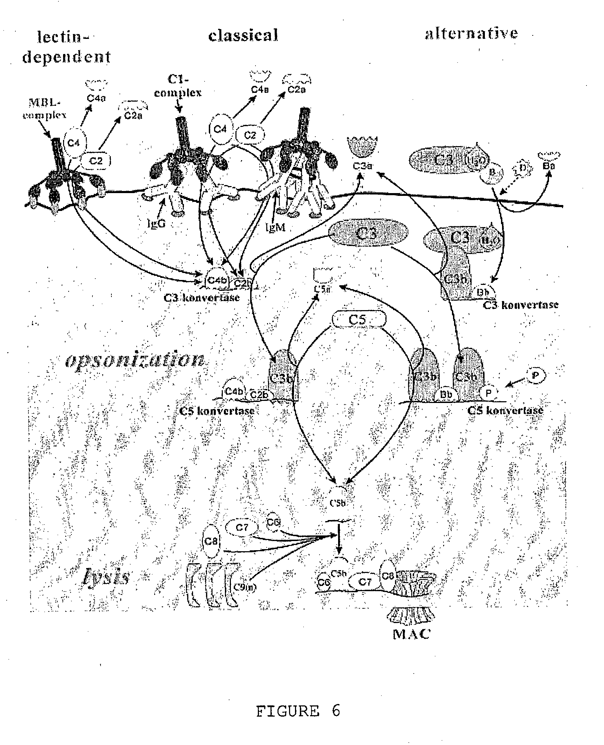

Flow sheet of the activation of complement system is shown in Figure 6.

Throughout the present application, antibody, antigen, antigen array and detecting agents are used for the simplicity of description (see definitions above).

The present invention relies on the ability of antigen recognition molecules, primarily antibodies, to activate the complement system in the sample tested, upon binding to elements of an antigen array.

This is in contrast to the method described in U.S. Patent Application US 2006/0263837 where the ability of C1q, the first component of complement, to bind to engaged antibodies printed as antibody arrays is utilized. Whereas Patent Application US 2006/0263837 employs C1q as a detection agent, irrespective of the sample tested, the present invention employs the functional complement system in the biological sample tested, thereby the information gained relates to antigen recognition properties and functional consequences in the organism from which the sample was taken.

It is well appreciated that immunoassays carried out with either antigen or antibody adsorbed in an indexed fashion to solid microporous surface have already been in use (EP 0063810). The property of antigen-bound antibodies to bind complement proteins, e.g. C1q, can be utilized for detecting immune complexes on such arrays.

Wahrmann et al. (Wahrmann et al., Human Immunology, 2005, 66(5): 526-534) discloses a method for the qualitative and quantitative analysis of complement activation, wherein covalently bound C4 fragments are detected with a labelled detecting agent and wherein micro-beads coated with antigens are used.

The present invention, in contrast to such assays, aims to integrate all pathways of complement activation in the tested sample, under assay conditions that allow complement activation to take place. Thus, instead of using complement proteins merely as detection agents, it assesses complement activation itself in the sample. Sample collection, storage and application to the array must be done consistent with general criteria for complement activity measurements.

The present invention detects products of complement activation, i.e. C3 or C4 fragments, as opposed to C1q. In accordance, the present invention incorporates control and reference materials that allow quantitation and inter-assay comparisons of complement deposition on the arrays.

In one preferred embodiment of the present invention, there is provided an antigen array system for detecting complement components captured on the antigen array. The system comprises an antigen array of two or more antigens, reference materials, and at least one detecting agent that is able to recognize complement component C3 or C4 in a covalently bound form.

An antigen array is an ordered spatial arrangement of two or more antigens on a physical substrate. Row and column arrangements are preferred due to the relative simplicity in making and assessing such arrangements but any arrangement can be selected by the user. The most common form of antigen arrays is that antigens are arrayed on a glass slide at high density. A sample containing recognition molecules and an intact complement system is passed over the array and the bound complement fragments are detected after washing.

An ordered, indexable arrangement of antigens can also be obtained by linking the antigens to microparticles of different sizes and/or different fluorescent properties. In this case - as opposed to the spatial arrangement - light scattering and/or fluorescent intensities of the particles serves to establish an array of one or more dimensions.

Antigens of an antigen array are preferably printed onto a solid support. Amongst the large number of solid-support materials applicable for the production of antigen arrays, those with great chemical resistance against solvents, good mechanical stability, low intrinsic fluorescence properties, flexibility of being readily functionalized are preferable. An additional important requirement in our case is that the material used does not activate complement per se. Certain polymeric substances, e.g. Sepharose beads, are known to activate complement and should not be used. Examples of well known solid supports include nitrocellulose, glass, silica, synthetic polymers, dextran, nylon, etc. Those skilled in the art will know of other suitable solid support for binding antigens, or will be able to ascertain such, using routine experimentation.

Antigens may be immobilized onto a support surface either by chemical ligation through a covalent bond or noncovalent binding. There are many known methods for covalently immobilizing proteins onto a solid support (Lam and Renil, Current Opinion in Chemical Biology, 2002, 6:353-358).

Antigens may be attached to various kinds of surfaces via diffusion, adsorption/absorption, covalent cross-linking and affinity. Antigens may be directly spotted onto plain glass surface. Antigen coupling to an indexable array of beads is carried out via identical techniques. To preserve the structure of antigens the spotting is carried out in a humidity-controlled environment. Additionally, bead arrays can be stored in the form of a suspension.

The surface of a substrate may be modified to achieve better binding capacity. For example, the glass surface may be coated with a thin nitrocellulose membrane or poly-L-lysine such that antigens can be passively adsorbed to the modified surface through non-specific interactions. In addition, streptavidin may be arrayed onto solid surfaces for capture of biotinylated molecules.

Antigen arrays can be fabricated by the transfer of antigens onto the solid surface in an organized high-density format followed by chemical immobilization. The techniques for fabrication of an array include, but are not limited to, photolithography, ink jet and contact printing, liquid dispensing and piezoelectrics. The patterns and dimensions of antigen arrays are to be determined by each specific application. The size of each antigen spot may be easily controlled by the users.

As discussed above, the present invention takes advantage of the generation of chemically reactive molecules of the activated complement system. The detecting agent of the present invention is capable of marking the covalently bound complement components, on the antigens of the array that activate the complement system. The nature of the detecting agent is not important for the present invention as long as the detecting agent is applicable for the present invention.

In one preferred embodiment, the present invention provides the detecting agents as antibodies specific for complement C3 or C4 fragments. Alternative detecting agents are soluble receptors for complement C3 or C4, which preferentially bind the covalently bound degradation fragments.

In addition, labeled, functional C3 or C4 molecules can be added to the sample prior to applying the sample to the antigen array. The labeled C3 or C4 molecules can then function as active components of the cascade and bind covalently to the activating antigens.

When the antigen array is contacted with a sample (see definitions above), the formation of antigen - antigen recognition molecule complexes can be performed under a variety of conditions. In general physiological pH, salt concentrations and temperatures that favor the activation of the complement system will be used.

The readout of the detecting agents bound to complement C3 or C4 in an antigen array can take up many forms. Prior to description of the readout methods, it is to be appreciated that the recognition molecules in a sample and the C3 or C4 molecules can be simultaneously detected.For example, the antibodies in a sample can be detectably labeled (see definitons above) with fluorescent dyes or detected by specific secondary antibodies in combination with the C3 or C4 detecting agents, to simultaneously quantify and/or verify the binding of recognition molecule and activation of the complement cascade. Therefore, the detection methods for detecting agents may be compatible with other immunoassays as users desire. Detection of C3 and C4 fragments is not equivalent but has different biological meaning. Measurement of one or both of these complement proteins can be required depending on the nature and purpose of the immunoassay.

Fluorescence detection methods are generally the preferred detection method because they are simple, safe and extremely sensitive and can have very high resolution with the possibility of multiplex detection. Typically, an antigen array is either directly probed with a fluorescent detecting molecule or in two steps by first using a tagged probe (e.g., biotin), which can then be detected in a second step using a fluorescently labeled affinity reagent (e.g., streptavidin).

A biotin labeled target can be detected by gold-conjugated streptavidin with silver enhance solution so that the resultant black image of microarray spots can be easily detected with a commercial CCD camera.

The detecting agents and anti-detecting agent antibodies may be attached by the 5' end of an oligonucleotide primer. Then the signals may be significantly increased by Rolling circle amplification (RCA).

C3 or C4 that binds to the antigen array can be detected using any means known in the art. In some embodiments, the C3 or C4 is labeled, using any methods known in the art. For example, C3 may be labeled with one or more labeling moieties including compositions that can be detected by photochemical, spectroscopic, biochemical, immunochemical, chemical, optical, electrical, bioelectronic, etc. means. For example, useful protein labels include radiolabels (e.g., 3H, 125I, 35S, 14C, or 32P), fluorescent dyes (e.g., fluorescein isothiocyanate, Texas red, rhodamine, and the like); electron-dense reagents, enzymes (e.g., LacZ, CAT, horse radish peroxidase, alkaline phosphatase and others, commonly used as detectable enzymes, either as marker gene products or in an ELISA); biotin, dioxigenin, or haptens and proteins for which antisera or monoclonal antibodies are available. A wide variety of labels and conjugation techniques are known and generally applicable to the present invention for the labeling of proteins, the only restriction being that C3 or C4 must remain functionally active.

Analysis of C3 or C4 bound to the antigen arrays can be quantitative, semi-quantitative or qualitative. "Detect" refers to identifying the presence, absence and/or amount of molecule to be detected. "Absence" of binding, and "lack of detection of product" as used herein includes insignificant levels. Reference materials are used for assessing the presence and functionality of complement C3 and C4 molecules in the tested sample and allow for quantitative inter-assay comparisons to be made. These reference materials are printed on the array along with the antigens. Reference materials include capture molecules with affinity to complement C3 or C4, purified standard C3 or C4 proteins and obligatory complement activating substances. Capture of intact C3 or C4 molecules on the array provides a system for confirming the presence of these molecules in the tested sample. Printing purified C3 or C4 with known concentrations allows quantitative measurements to be made. Obligatory complement activators are of three classes: classical pathway activators (e.g. aggregated immunoglobulin), lectin pathway activators (e.g. mannan) and alternative pathway activators (e.g. properdin). Complement activators serve to verify that all three pathways are active in the tested serum.

It is to be appreciated that the detection of complement activation on an antigen array in combination with the detection of antibody binding provides an alternative to isotype (class, subclass) determination of the bound antibodies. The isotype of an antibody strongly influences its complement activating capacity, thus this functional assay can replace and even surpass isotype characterizations on antigen arrays.

Characterization of immune responses against parasitic, bacterial, viral and prion infections or following a vaccination is of crucial importance in the clinical practice. Antigen microarrays have been successfully applied for serodiagnosis of infectious diseases (Mezzasoma et al., Clin. Chem. 2002, 48:121-130). In another embodiment, the disclosed methods are useful for evaluating the nature of the immune response with respect to the complement activating properties of the induced antibodies. Complement activating properties often reflect protective characteristics of microbe specific antibodies, thus, immunity against particular microbes can be assessed by the present invention. In addition to identifying serotypes of microbes, against which immunity has evolved, complement activation by components of microbes can also give insight on the nature of the response. Therefore printing various components of a microbe as elements of an array can provide a profile of the individual's humoral immunity with respect to that particular microbe. In a similar fashion, cancer cells and components thereof can be used as antigens, in order to assess immunity against tumours.

The systems and methods disclosed herein can be used in methods of diagnosing and monitoring particular autoimmune disorders. The generation of autoantigen microarrays have been described (Robinson et al., Nat. Med. 8:295-301). For example, in a diagnostic kit, a collection of autoantigens specific for one or more disorders can be arrayed and contacted with a body fluid containing autoantibodies whose presence or absence and complement activating properties would indicate a particular disorder or severity of the disease.

The advantage of using a method for identifying complement activating antigens is that functionality of the arraybound antibodies is assessed as well, improving sensitivity, specificity and differential diagnostic power of the analysis.

As opposed to most solid-phase immunoassays, where samples are diluted from hundred- up to several thousand-times, samples are either undiluted or minimally diluted (ten times) in the present description. This dilution factor allows all complement pathways to function and, additionally, mimics in vivo situations more closely. Results obtained with the present invention are therefore a very close approximation of the events initiated upon antigen coming into contact with fluids in the body.

Polyreactive antibodies with low binding affinity against a number of host and foreign antigens are found in the circulation normally. These are mostly IgM and also IgG antibodies, many belong to the so called natural antibody group and are produced predominantly by the so called B-1 cells of the B lymphocyte population. It is because of the polyreactivity of these antibodies that sensitive immunoassays are usually carried out from diluted serum samples, reducing background signal by the elimination of low-affinity binders. Thus, applying undiluted or minimally diluted serum samples to antigens is always expected to result in IgM binding. Since IgM is the most potent activator of complement, complement activation is also expected to take place. Additionally, complement activation can also proceed via the alternative pathway when regulator molecules are absent from a surface. In accordance, all foreign materials need to be tested for biocompatibility with respect to complement activating properties.

Notwithstanding this reactivity of the complement system, our technology generates information that relies on the presence of biologically relevant recognition molecules, producing biologically meaningful information, as opposed to general background noise.

In a preferred embodiment, the results obtained with the present invention are expressed as a two-dimensional space, where the coordinates of a particular antigen of the array are derived from the simultaneous measurement of antibody binding and complement C3 or C4 deposition on the antigen. In general, most variability in complement activation is expected to derive from the presence/absence and quality of antibodies specific for that particular antigen. Such a two-dimensional representation therefore renders a particular C3 or C4 value to a given antibody binding value for each antigen on the array. This kind of representation, termed two-dimensional immune profile, helps the biological and medical diagnostic interpretation of data. Antibodies belonging to different classes and subclasses have distinct complement activating properties (Immunobiology 5th ed., Janeway, Charles A.; Travers, Paul; Walport, Mark; Shlomchik, Mark; New York and London: Garland Science; c2001). In a given biological sample antibodies belonging to different classes and subclasses will bind to the same antigen. It is of medical and immunological relevance to determine the relative proportion of these antibodies. One partial solution is the isotype specific detection of antibodies on antigen arrays (Graham KL, Vaysberg M, Kuo A, Utz PJ.; Proteomics. 2006 Nov;6(21):5720-4). Due to limitations in multicolor detection this approach cannot provide an all-inclusive result. As an alternative solution, integrated functions of the bound antibodies can be measured, such as complement activation.

The present invention provides a system which is suitable for the integrated functional measurement of antigen specific antibodies. In one preferred embodiment IgG binding and C3 fragment deposition data are used to obtain a two-dimensional representation of antigen recognition by the organism from which the sample was taken.

It is to be appreciated that many conventional procedures are not described herein including blocking and washing steps in performing the methods of the present invention. These procedures are well known to those in the art.

Summarising the advantages of the present invention:

The basis of the present invention is that antigens on an antigen array can initiate complement activation both by antibody-dependent and independent way.

The systems and methods disclosed herein can be used in methods of diagnosing and monitoring particular autoimmune disorders and infections. This new diagnostic method utilizing an antigen array for simultaneously identifying different antigens capable of activating the complement system, in a quantitative fashion solves a particular problem.

The present invention relates to multiplex immunoassays utilizing antigen arrays, and more particularly to methods for qualitative and quantitative detection of antigens activating complement in a biological sample, via the measurement of complement components deposited on antigen arrays.

The invention employs the functional complement system in the biological sample tested, thereby the information gained relates to antigen recognition properties and functional consequences in the organism from which the sample was taken and relies on the ability of antigen recognition molecules, primarily antibodies to activate the complement system in the sample tested, upon binding to elements of an antigen array.

### DESCRIPTIONS OF FIGURES 1-6

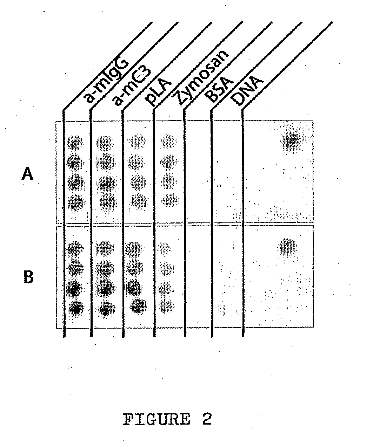

Figure 1. Detection of complement activation on antigen array
   The first step of the method is the generation of an array of indexed antigens and reference materials. Upon application of the biological sample complement can be activated by any or any combination of the three known pathways, such as classical, lectin or alternative pathway. After the removal of unbound substances deposited C3 (or C4) can be detected.
   Ab antibody
   Ag antigen
   CCC complement convertase complex
   MBL mannose binding lectin
   C3b degradation product generated from C3 by CCC
   C3bBb C3 convertase of the alternative pathway
Figure 2. Complement activation by DNA specific antibody on an antigen array
   A sample without DNA specific antibody
   B sample with DNA specific antibody
   a-mIgG goat anti-mouse IgG capture antibody
   a-mC3 goat anti-mouse complement C3 capture antibody
   pLA bacterial superantigen fusion protein which activates complement via antibody binding
   zymosan cell wall component of yeast, activating complement via the lectin and alternative pathway
   BSA bovine serum albumin, negative control
   DNA deoxyribonucleic acid, double stranded
   The spot in the upper right corner is an orientation spot which helps location and identification of features of the array.

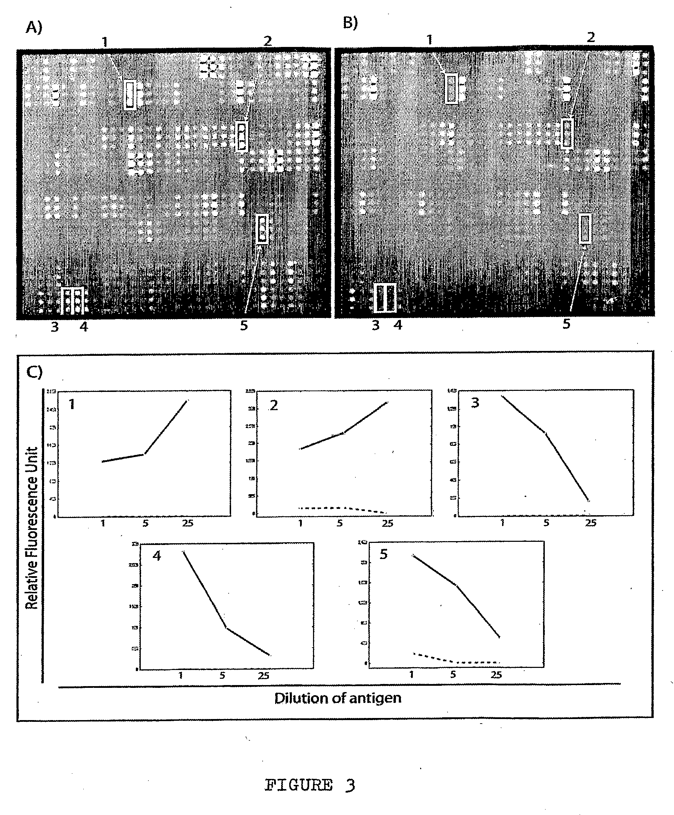

Figure 3. Comparison of serum profiles of an SLE patient and a control subject
   A) SLE patient, image of microarray
   B) control subject, image of microarray
      Complement C3 is represented by green, IgM antibodies by red; when both are bound mixtures of the two colors are generated.
      Indicated antigens are
      1 double stranded DNA
      2 Nucleosome
      3 Ro (SSA) antigen
      4 La (SSB) antigen
      5 Cytomegalovirus
   C) Quantitative results of the above experiment
      Solid blue lines represent the SLE patient, dashed red lines the control subject; relative fluorescent intensities measured from the indicated 1-, 5- and 25-times diluted antigens are shown
      SLE systemic lupus erythematosus, autoimmune condition
Figure 4. Representation of concurrent C3 deposition and IgG binding to a series of antigen dilutions
   Joined black boxes represent results from an individual, the three boxes being three, 5-fold different dilutions of the antigen. Results from five animals are shown. Values are relative fluorescence units.
Figure 5. Images of complement C3 and C4 deposition from the same serum sample on an antigen array.
Figure 6. Flow sheet of the activation of complement system.

### EXAMPLES

The following examples are offered by way of illustration and not by way of limitation.

### Example 1 (figure 2) Measurement of antibody dependent complement activation by an autoantigen

Low density nitrocellulose arrays comprising the indicated antigens were incubated in normal, healthy mouse serum after treatment with physiological saline solution (A) or with an identical solution supplemented with 8 micrograms of DNA specific monoclonal antibody. 600 microliters of serum were dropped on the arrays and kept in a humidified atmosphere at 37 degrees Celsius for 60 minutes. The arrays were subsequently washed three times in phosphate buffered saline solution, then goat anti-mouse C3 antibody labeled with FITC, diluted 1:5000, was dropped on the arrays. Arrays were agitated for 30 minutes then washed again three times and scanned on a Typhoon Trio+ scanner (GE Healthcare).

Figure 2. shows fluorescent images of the arrays captured with settings ideal for fluorescein detection.
- a-mIg: Anti-mouse IgG activates complement in both sera by capturing IgG and initiating the classical pathway of complement.
- a-mC3: Anti-mouse C3 captures C3 molecules from both sera, these are subsequently detected.
- pLA is a bacterial superantigen fusion protein which binds and activates immunoglobulins and thereby complement.
- Zymosan is a cell wall component of baker's yeast which activates complement via the lectin and alternative pathways in both sera.
- Bovine serum albumin serves as an irrelevant protein which does not activate complement.
- DNA only activates complement in the serum when the array was pretreated with a DNA specific monoclonal antibody, proving the specificity of the method.

### Example 2 (figure 3) Comparison of control and autoimmune serum profiles

85 different types of antigens (autoantigens, microbial components, control proteins) were spotted in three different concentrations onto nitrocellulose arrays for the determination of a broader immunoprofile of a control and a patient suffering from systemic autoimmune disease. Protein arrays were incubated in 5 times diluted human serum at 37°C for 1 hour, conditions suitable for both antibody binding and complement activation. Sera were diluted in a buffer containing calcium and magnesium ions to allow complement activation to take place. Protein chips were washed, then incubated in 5000x diluted Alexa-647 conjugated anti-human IgM (Southern Biotech) and 2500 times diluted FITC labeled anti-human C3 (MP Biomedicals) antibodies for 30min. Protein arrays were washed again and bound IgM and the deposited complement C3b and C3d fragments were measured by Typhoon Trio+ scanner (GE Healthcare).

Figure 3 shows immunoprofiles of the autoimmune (A) and control (B) patient. In the false colored pictures green represents complement activation while red stands for bound IgM antibodies. Five antigens are boxed and numbered(1- dsDNA, 2- Nucleosome, 3- Ro (SSA) antigen, 4- La (SSB) antigen, 5-Cytomegalovirus) to allow comparison by the naked eye. Figure 3C shows the signal intensity in relative fluorescence units of bound C3 fragment, as a function of the concentration of spotted antigen (autoimmune patient - solid blue, control-dashed red) in the case of the five chosen antigens. It is readily visible that autoantigens induce significant complement activation,only in the autoimmune patient's serum. Additionally, positive reactions with cytomegalovirus indicates past or present infection with this virus.

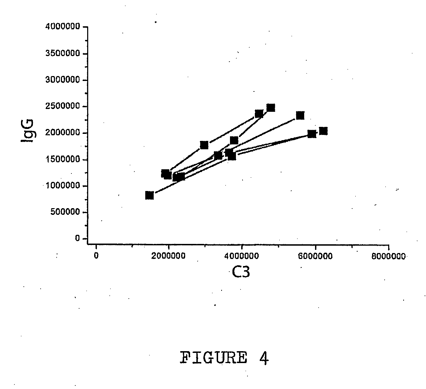

### Example 3 (figure 4) Representation of concurrent C3 deposition and Ig binding measurements

Mice (C57/B6 strain) were immunized with a model antigen TNP-KLH (Tri-nitro-phenyl conjugated to Keyhole Limpet Hemocyanin) in the presence of Al(OH)₃ adjuvant. The result of immunization was tested with the help protein arrays where serial dilutions of TNP-BSA (Tri-nitro-phenyl conjugated to Bovine Serum Albumin) and control proteins were spotted onto nitrocellulose-coated glass slides. Protein arrays were incubated in undiluted mouse serum at 37°C for 1 hour. Following washing, protein chips were incubated in FITC labeled goat-anti mouse C3 antibody and Alexa-647 conjugated goat anti-mouse IgG antibody for 30min. Fluorescence signal was detected by Typhoon Trio+ scanner (GE Healthcare). The graph of Figure 4 was generated by plotting C3 signals from a particular spot of given antigen density against the IgG antibody signal from the same spot. The three different antigen concentrations are represented by black boxes joined by lines, each line represents results from an individual animal.

With the immunization scheme used, both IgG binding and C3 fragment deposition can be detected on the antigen, suggesting that TNP specific antibodies capable of activating complement were induced.

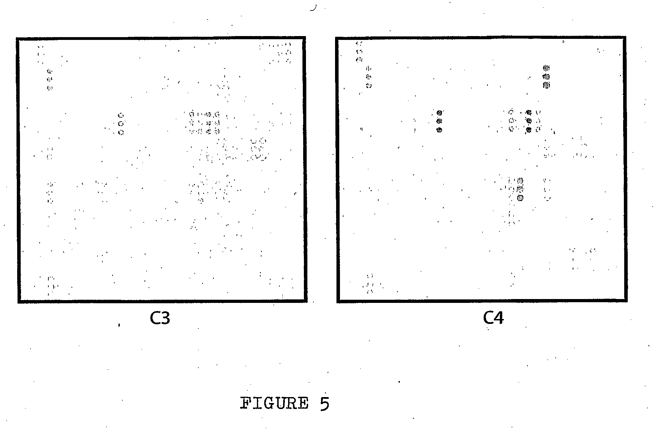

### Example 4 (figure 5) Comparison of C3 and C4 deposition patterns

85 different types of antigens (autoantigens, microbial components, control proteins) were spotted in three different concentrations onto nitrocellulose arrays for the comparison of C3 and C4 deposition patterns. Protein arrays were incubated in the same 5 times diluted human serum at 37°C for 1 hour, conditions suitable for complement activation. Protein chips were washed, then incubated either in 2500 times diluted FITC labeled anti-human C3 (MP Biomedicals) or in 2500 times diluted FITC labeled anti-human C4 (MP Biomedicals) antibodies for 30min. Protein arrays were washed again and the deposited complement C3b/C3d or C4b fragments were measured by Typhoon Trio+ scanner (GE Healthcare).

Figure 5 shows patterns of C3 and C4 deposition. Although C4 activation precedes C3 activation via the classical and lectin pathways, correlation between C4 and C3 deposition is not strong, suggesting that different pathways contribute to the generation of complement deposition profiles to a different extent. This result strengthens that both C3 and C4 deposition is measurable on antigen arrays and that their measurement provides distinct biological and medical values.

While the present invention has been described with reference to particular embodiments, it will be understood that the embodiments are illustrative. Accordingly, the scope of the present invention is described by the appended claims and is supported by the foregoing description.

## Claims

1. A method for the qualitative and quantitative analysis of complement activation on an antigen array, wherein the antigen array comprises at least two antigens, and wherein covalently bound C3 and/or C4 fragments are detected with a labelled detecting agent, ***characterised by*** the following steps:
Using an antigen microarray as the antigen array; wherein antigens and reference materials are printed side-by-side, as spots on the microarray, at high spatial density;
contacting the antigen microarray with a sample containing both at least one molecule that may be captured by the antigens disposed on the antigen microarray and a functional complement system; allowing complement activation to take place in a single reaction chamber per sample on the microarray, resulting in localized, antigen-specific deposition of C3 and/or C4 fragments;
measuring bound C3 and/or C4 fragments covalently bound to the microarray as a result of complement activation by that particular antigens using a labelled detecting agent.

2. The method of claim 1, wherein the detecting agent is directly labelled, functionally active C3 and/or C4 molecule, capable of covalently binding to the antigens on the microarray.

3. The method of claim 1, wherein the antigens of the antigen microarray are parasites, microbes, viruses, prions or components thereof.

4. The method of claim 1, wherein the antigens are selected from the group consisting of proteins, peptides, glycoproteins, lipoproteins, lipids, glycolipids, nucleic acids, carbohydrates, small molecules, and allergens or autoantigens.

5. The method of claim 1, wherein the sample includes a clinical sample, serum, plasma, biological fluid, and cultured cells, cell supernatants, cell lysates, or a pure or enriched sample derived from any of these.

6. The method of claim 1, wherein the detecting agent is an antibody or portions or fragments thereof.

7. The method of claim 1, wherein the detecting agent is a soluble receptor protein or portions or fragments thereof.

8. The method of claim 1, wherein the detecting agent is a scaffold protein, nucleic acid aptamer or other affinity reagent obtained from libraries by molecular evolution and affinity screening.

9. The method of claim 1, wherein the microarray is a two dimensional microarray on a solid surface.

10. The method of claim 1, wherein the microarray contains reference material that capture complement C3 or C4 molecules and/or activate the complement system and/or are purified C3 or C4 proteins themselves.

11. A method of claim 1 for measurement of antibody dependent complement activation by an autoantigen or for comparison of control and autoimmune serum profiles, or for representation of concurrent C3 deposition and Ig binding measurements, or for comparison of C3 and C4 deposition patterns, ***characterised by*** the following steps:
Low density nitrocellulose microarrays comprising the indicated antigens are contacted with serum samples and kept in a humidified atmosphere at 37 degrees Celsius, then the microarrays are subsequently washed in buffered saline solution, then fluorescently directly labelled C3 or C4 specific antibody, diluted to give optimal signal to noise ratio, is contacted with the microarrays, agitated, then the microarray is washed again and scanned on a scanner to give a microarray where the at least one antigen that captured the molecule capable of complement activation is detected.

## Patentansprüche

1. Verfahren für die qualitative und quantitative Analyse der Komplementaktivierung auf einem Antigen-Array, wobei der Antigen-Array mindestens zwei Antigene aufweist und wobei kovalent gebundene C3- und/oder C4-Fragmente mit einem markierten Nachweismittel nachgewiesen werden, **gekennzeichnet durch** die folgenden Schritte:
Verwendung eines Antigen-Mikroarrays als Antigen-Array, wobei Antigene und Referenzmaterialien nebeneinander als Punkte auf dem Mikroarray mit einer hohen räumlichen Dichte aufgetragen sind;
Inkontaktbringen des Antigen-Mikroarrays mit einer Probe, die sowohl mindestens ein Molekül, das **durch** die Antigene, die auf dem Antigen-Mikroarray angeordnet sind, eingefangen wird, als auch ein funktionales Komplementsystem enthält;
Ermöglichen, dass die Komplementaktivierung in einer einzelnen Reaktionskammer des Mikroarrays pro Probe stattfindet, was zu einer lokalisierten, antigenspezifischen Ablagerung von C3- und/oder C4-Fragmenten führt;
Messung der gebundenen C3- und/oder C4-Fragmente, die als Ergebnis der Komplementaktivierung **durch** bestimmte Antigene kovalent an den Mikroarray gebunden sind, unter Verwendung eines markierten Nachweismittels.

2. Verfahren nach Anspruch 1, wobei das Nachweismittel ein direkt markiertes, funktionell aktives C3-und/oder C4-Molekül ist, das in der Lage ist, kovalent an die Antigene auf dem Mikroarray zu binden.

3. Verfahren nach Anspruch 1, wobei die Antigene auf dem Antigen-Mikroarray von Parasiten, Mikroben, Viren, Prionen oder Komponenten davon sind.

4. Verfahren nach Anspruch 1, wobei die Antigene ausgewählt sind aus der Gruppe, die aus Proteinen, Peptiden, Glykoproteinen, Lipoproteinen, Lipiden, Glykolipiden, Nukleinsäuren, Kohlenhydraten, kleinen Molekülen und Allergenen oder Autoantigenen besteht.

5. Verfahren nach Anspruch 1, wobei die Probe eine klinische Probe, Serum, Plasma, ein biologisches Fluid und kultivierte Zellen, Zellüberstände, Zelllysate oder eine reine oder angereicherte Probe umfasst, die von irgendeinem von diesen abgeleitet wurde.

6. Verfahren nach Anspruch 1, wobei das Nachweismittel ein Antikörper ist oder Abschnitte oder Fragmente davon sind.

7. Verfahren nach Anspruch 1, wobei das Nachweismittel ein lösliches Rezeptorprotein oder Abschnitte oder Fragmente davon sind.

8. Verfahren nach Anspruch 1, wobei das Nachweismittel ein Gerüstprotein, ein Nukleinsäure-Aptamer oder ein anderes Affinitätsreagenz ist, das aus Bibliotheken durch molekulare Evolution und Affinitätsscreening erhalten wurde.

9. Verfahren nach Anspruch 1, wobei der Mikroarray ein zweidimensionaler Mikroarray auf einer festen Oberfläche ist.

10. Verfahren nach Anspruch 1, wobei der Mikroarray Referenzmaterial enthält, das C3- oder C4-Komplementmoleküle einfängt und/oder das Komplementsystem aktiviert und/ selber gereinigte C3- oder C4-Proteine sind.

11. Verfahren nach Anspruch 1 zur Messung der antikörperabhängigen Komplementaktivierung durch ein Autoantigen oder zum Vergleich von Kontroll- und Autoimmunserum-Profilen oder zur Darstellung gleichzeitiger C3-Ablagerungs- und Ig-Bindungsmessungen oder zum Vergleich von C3- und C4-Ablagerungsmustern, **gekennzeichnet durch** die folgenden Schritte:
Nitrozellulose-Mikroarrays niedriger Dichte, welche die angegebenen Antigene aufweisen, werden mit den Serumproben in Kontakt gebracht und bei 37 °C in einer angefeuchteten Atmosphäre gehalten, anschließend werden die Mikroarrays daraufhin in gepufferter Kochsalzlösung gewaschen, danach direkt fluoreszenzmarkierte C3- oder C4-spezifische Antiköper, die verdünnt wurden, um das optimale Signal-Rausch-Verhältnis zu ergeben, mit den Mikroarrays in Kontakt gebracht, geschüttelt, anschließend der Mikroarray nochmals gewaschen und auf einer Abtasteinrichtung abgetastet, um einen Mikroarray zu ergeben, in dem das mindestens eine Antigen nachgewiesen wird, welches das Molekül einfing, das zur Komplementaktivierung in der Lage ist.

## Revendications

1. Procédé d'analyse qualitative et quantitative de l'activation du complément sur un réseau d'antigènes, dans lequel le réseau d'antigènes comprend au moins deux antigènes, et dans lequel des fragments C3 et/ou C4 liés par liaison covalente sont détectés avec un agent marqué de détection, **caractérisé par** les étapes suivantes :
l'utilisation d'un micro-réseau d'antigènes jouant le rôle du réseau d'antigènes ; dans lequel des antigènes et des substances de référence sont imprimées côte à côte sous la forme de spots sur le micro-réseau à une densité spatiale élevée ;
le contact du micro-réseau d'antigènes avec un échantillon contenant à la fois au moins une molécule qui peut être capturée par les antigènes disposés sur le micro-réseau d'antigènes et un système fonctionnel du complément ; le fait de laisser l'activation du complément avoir lieu dans une unique chambre réactionnelle par l'échantillon sur le micro-réseau, pour obtenir un dépôt localisé spécifique d'un antigène de fragments C3 et/ou C4 ;
la mesure des fragments C3 et/ou C4 liés par liaison covalente au micro-réseau du fait de l'activation du complément par ces antigènes particuliers à l'aide d'un agent marqué de détection.

2. Procédé selon la revendication 1, dans lequel l'agent de détection est une molécule C3 et/ou C4 fonctionnellement active et marquée directement capable de se lier par liaison covalente aux antigènes sur le micro-réseau.

3. Procédé selon la revendication 1, dans lequel les antigènes du micro-réseau d'antigènes sont des parasites, des microbes, des virus, des prions ou leurs composants.

4. Procédé selon la revendication 1, dans lequel les antigènes sont choisis dans le groupe constitué des protéines, des peptides, des glycoprotéines, des lipoprotéines, des lipides, des glycolipides, des acides nucléiques, des glucides, des petites molécules et des allergènes ou sont des auto-antigènes.

5. Procédé selon la revendication 1, dans lequel l'échantillon comprend un échantillon clinique, du sérum, du plasma, un fluide biologique et des cellules cultivées, des surnageants cellulaires, des lysats cellulaires, ou un échantillon pur ou enrichi dérivé de l'un quelconque de ceux-ci.

6. Procédé selon la revendication 1, dans lequel l'agent de détection est un anticorps ou des parties ou des fragments de celui-ci.

7. Procédé selon la revendication 1, dans lequel l'agent de détection est une protéine de récepteur soluble ou des parties ou des fragments de celle-ci.

8. Procédé selon la revendication 1, dans lequel l'agent de détection est une protéine de structure, un aptamère d'acides nucléiques ou un autre réactif d'affinité obtenu à partir de banques par évolution moléculaire et criblage par affinité.

9. Procédé selon la revendication 1, dans lequel le micro-réseau est un micro-réseau bidimensionnel sur une surface solide.

10. Procédé selon la revendication 1, dans lequel le micro-réseau contient une substance de référence qui capture des molécules C3 ou C4 du complément et/ou active le système du complément et/ou est une protéine C3 ou C4 purifiée elle-même.

11. Procédé selon la revendication 1, permettant de mesurer l'activation du complément dépendant d'un anticorps par un auto-antigène ou de comparer des profils de sérum auto-immun et témoin, ou de représenter des mesures de dépôt de C3 et de liaison d'Ig concourants, ou de comparer des motifs de dépôt de C3 et de C4, **caractérisé par** les étapes suivantes :
des micro-réseaux de cellulose basse densité comprenant les antigènes indiqués sont mis en contact avec des échantillons de sérum et maintenus sous une atmosphère humidifiée à 37 degrés Celsius, puis les micro-réseaux sont lavés avec une solution saline tamponnée, puis un anticorps directement marqué par fluorescence et spécifique de C3 ou C4, dilué pour donner un rapport signal sur bruit optimal, est mis en contact avec les micro-réseaux, agité, puis le micro-réseau est à nouveau lavé et analysé par balayage avec un analyseur pour donner un micro-réseau sur lequel le au moins un antigène qui a capturé la molécule capable d'activer le complément est détecté.
